# EUROPEAN PATENT APPLICATION

(11) **EP 4 636 776 A1**
(43) Date of publication of application: **22.10.2025**
(21) Application number: 24171350.2
(22) Date of filing: 19.04.2024
(51) Int. Cl.: G16H 20/13, G16H 40/60

(54) **METHOD AND SYSTEM FOR EXTRACTING INFORMATION FROM MEDICATION SACHETS**

(71) Applicant: Evondos Oy, 24240 Salo (FI)
(72) Inventor: RANNISTO, Joni, 11130 Riihimäki (FI); KORKEE, Markus, 38250 Sastamala (FI)
(74) Representative: Berggren Oy

(57) **Abstract**

The present invention relates to a method and system for extracting information from medication sachets (201, 301, 401). In the present invention, locations (208, 209) of dispense date (203) and dispense time (204) on a medication sachet (201) are determined and, by using these locations (208, 209), the dispense date (303) and dispense time (304) for another medication sachet (301) is determined. The invention also relates to a medication dispenser (400).

## Description

### TECHNICAL FIELD OF THE INVENTION

The present invention relates to a method and system for extracting information from medication sachets according to the preambles of the appended independent claims. The invention also relates to a medication dispenser.

### BACKGROUND OF THE INVENTION

Various medication dispensers are known for automated dispensing of medications to a patient. Many of these medication dispensers use strips of prepackaged medication sachets, wherein each medication sachet contains the medications that the patient should take at a specific (i.e. prescribed) date and time. The medication dispenser separates the medication sachets from the strip and delivers them to the patient one sachet at a time.

The medication sachets are provided with patient and medication related information, such as a patient identifier (e.g. name and/or identification number), medication name(s) and dosage(s), and dispense (i.e. taking) date and time. This information is printed using a specific layout on a surface of the medication sachet or on a label that is attached on the medication sachet. The layout defines the locations for each type of information. The medication dispenser uses the patient and medication related information that is printed on the medication sachets for the purpose of dispensing medications to a patient.

A problem associated with the known medication dispensers is that, in order to extract information from medication sachets, the medication dispenser must know the layout according to which the information has been printed on the medication sachets. If the layout is not known, the medication dispenser cannot dispense medications to the patient.

### OBJECTIVES OF THE INVENTION

It is the main objective of the present invention to reduce or even eliminate the prior art problems presented above.

It is an objective of the present invention to provide a method and system for extracting information from medication sachets. In more detail, it is an objective of the invention to provide a method and system enabling to extract information from medication sachets without knowing the layout according to which the information has been printed on the medication sachets. It is a further objective of the invention to provide a method and system enabling to extract information from medication sachets easily and accurately.

It is also an objective of the present invention to provide a medication dispenser for dispensing medications to a patient. In more detail, it is an objective of the invention to provide a medication dispenser enabling to dispense medications without knowing the layout according to which the information has been printed on the medication sachets.

In order to realise the above-mentioned objectives, the method and system according to the invention are characterised by what is presented in the characterising portions of the appended independent claims. Advantageous embodiments of the invention are described in the dependent claims.

### DESCRIPTION OF THE INVENTION

A method according to the invention for extracting information from medication sachets comprises:
- capturing an image of a first medication sachet,
- identifying from the image of the first medication sachet a text string corresponding to a predefined dispense date of the first medication sachet, determining a location of said text string in the image of the first medication sachet and setting said location as a location of the dispense date,
- identifying from the image of the first medication sachet a text string corresponding to a predefined dispense time of the first medication sachet, determining a location of said text string in the image of the first medication sachet and setting said location as a location of the dispense time,
- capturing an image of a second medication sachet,
- identifying from the image of the second medication sachet a text string at the location of the dispense date and using said text string as a dispense date for the second medication sachet, and
- identifying from the image of the second medication sachet a text string at the location of the dispense time and using said text string as a dispense time for the second medication sachet.

The method according to the invention can be used in a medication dispenser for extracting information from medication sachets for the purpose of dispensing medications to a patient. The information on the medication sachets can be patient and medication related information, such as a patient identifier (e.g. name and/or identification number), medication name(s) and dosage(s), and dispense date and time. This information has been printed using a specific layout on the medication sachets, either directly on a surface of the medication sachet or on a label that is attached on the medication sachet. Each medication sachet contains the medications that the patient should take at a prescribed date and time. The medication sachets are preferably attached to each other so that they form a strip of medication sachets. The dispense date can be represented in various formats, such as DD/MM/YYYY, DD.MM.YYYY, DD-MM-YYYY, MM/DD/YYYY, MM.DD.YYYY, or MM-DD-YYYY, where DD is the day, MM is the month, and YYYY is the year, or DD Month YYYY, where DD is the day, Month is the name of a month or its abbreviation, and YYYY is the year. The dispense time can be represented in various formats, such as HH:MM, or HH:MM XM, where HH is the hours, MM is the minutes, and XM is either AM or PM. The dispense time can also be represented as a text that indicates the time of day, such as morning, afternoon, or evening.

The method according to the invention begins with the processing of a first medication sachet. The first medication sachet is preferably the medication sachet that is to be dispensed first from a strip of medication sachets to a patient. The processing of the first medication sachet begins with capturing an image of the first medication sachet. An imaging device such as a digital camera can be used for capturing the image. The captured image can be composed from one or more images taken of the first medication sachet. Then, a text string that corresponds to a predefined dispense date of the first medication sachet is identified from the image, a location of said text string in the image is determined and said location is set as a location of the dispense date; and a text string that corresponds to a predefined dispense time of the first medication sachet is identified from the image, a location of said text string in the image is determined and said location is set as a location of the dispense time. The text strings can be identified using optical character recognition (OCR), and the locations of the text strings can be determined relative to the other features of the medication sachet (such as edge).

The predefined dispense date and time of the first medication sachet can be stored in a memory located in a medication dispenser from which memory the dispense date and time is retrieved for the purpose of identifying text strings in the image. The dispense date and time of the first medication sachet can be fed into the memory at the same time a strip of medication sachets is inserted into a container of the medication dispenser. The dispense date and time of the first medication sachet can alternatively be received to the memory of the medication dispenser from a server over a communications network. The first medication sachet can be dispensed to a patient according to the predefined dispense date and time.

After the first medication sachet has been processed, the method according to the invention continues with the processing of a second medication sachet. The second medication sachet is preferably the next medication sachet in the strip of medication sachets. The processing of the second medication sachet begins with capturing an image of the second medication sachet. An imaging device such as a digital camera can be used for capturing the image. The captured image can be composed from one or more images taken of the second medication sachet. Then, a text string at the location of the dispense date is identified from the image and said text string is used as a dispense date for the second medication sachet; and a text string at the location of the dispense time is identified from the image and said text string is used as a dispense time for the second medication sachet. The second medication sachet can be dispensed to a patient according to the dispense date and time determined from the image of the second medication sachet.

After the second medication sachet has been processed, further medication sachets can be processed in a similar way compared to the second medication sachet.

An advantage of the method according to the invention is that information can be extracted from medication sachets without knowing the layout according to which the information has been printed on the medication sachets. Another advantage of the method according to the invention is that information can be extracted from medication sachets easily and accurately.

According to an embodiment of the invention the method comprises searching from the image of the first medication sachet a text string corresponding to a predefined patient identifier. The patient identifier can be any suitable identifier for identifying the patient, such as a name or an identification number of the patient. The predefined patient identifier can be stored in a memory located in a medication dispenser from which memory the patient identifier is retrieved for the purpose of searching a corresponding text string in the image. The patient identifier can be fed into the memory at the same time a strip of medication sachets is inserted into a container of the medication dispenser. The patient identifier can alternatively be received to the memory of the medication dispenser from a server over a communications network. By searching the predefined patient identifier from the image of the first medication sachet, it can be verified that the medications are correct for the patient.

According to an embodiment of the invention the method comprises, if a text string corresponding to the predefined patient identifier is not found, giving an alarm. The alarm can be a sound, a light or other visual signal that is given to the patient, or a message that is transmitted over a communications network to a caregiver or a healthcare person.

According to an embodiment of the invention the method comprises, if a text string corresponding to the predefined patient identifier is found, determining a location of said text string in the image of the first medication sachet and setting said location as a location of the patient identifier.

According to an embodiment of the invention the method comprises identifying from the image of the second medication sachet a text string at the location of the patient identifier, comparing said text string to the predefined patient identifier, and, if a match is not found, giving an alarm. By comparing the predefined patient identifier to the text string at the location of the patient identifier, it can be verified that the medications are correct for the patient.

According to an embodiment of the invention the steps of the method according to the invention are carried out in a medication dispenser.

According to an embodiment of the invention the method comprises transmitting from the medication dispenser to another medication dispenser one or more of the following types of information: the location of the dispense date, the location of the dispense time, and the location of the patient identifier.

The present invention also relates to a system for extracting information from medication sachets. The system according to the invention comprises:
- an imaging device configured to capture an image of a first medication sachet and an image of a second medication sachet, and
- a processing device configured to:
   - receive the image of the first medication sachet and the image of the second medication sachet from the imaging device,
   - identify from the image of the first medication sachet a text string corresponding to a predefined dispense date of the first medication sachet, determine a location of said text string in the image of the first medication sachet and set said location as a location of the dispense date,
   - identify from the image of the first medication sachet a text string corresponding to a predefined dispense time of the first medication sachet, determine a location of said text string in the image of the first medication sachet and set said location as a location of the dispense time,
   - identify from the image of the second medication sachet a text string at the location of the dispense date and use said text string as a dispense date for the second medication sachet, and
   - identify from the image of the second medication sachet a text string at the location of the dispense time and use said text string as a dispense time for the second medication sachet.

The system according to the invention can be used in a medication dispenser for extracting information from medication sachets for the purpose of dispensing medications to a patient.

The imaging device may comprise a digital camera for capturing images of the medication sachets. The imaging device may also comprise one or more light sources for illuminating the medication sachets from one or more directions.

The processing device can be implemented in hardware, software, or a combination of hardware and software components. Hardware components may comprise a processor for processing data and a storage medium for storing the data. Software components may be in the form of computer-readable program code stored in a computer-readable storage medium such as memory, mass storage device, or removable storage device. For example, a computer-readable medium may comprise computer-readable code for performing the function of a particular component. Likewise, computer memory may be configured to include one or more components, which may then be executed by a processor. Components may be implemented separately in multiple modules or together in a single module.

An advantage of the system according to the invention is that information can be extracted from medication sachets without knowing the layout according to which the information has been printed on the medication sachets. Another advantage of the system according to the invention is that information can be extracted from medication sachets easily and accurately.

According to an embodiment of the invention the processing device is configured to search from the image of the first medication sachet a text string corresponding to a predefined patient identifier.

According to an embodiment of the invention the processing device is configured to, if a text string corresponding to the predefined patient identifier is not found, give an alarm.

According to an embodiment of the invention the processing device is configured to, if a text string corresponding to the predefined patient identifier is found, determine a location of said text string in the image of the first medication sachet and set said location as a location of the patient identifier.

According to an embodiment of the invention the processing device is configured to identify from the image of the second medication sachet a text string at the location of the patient identifier, compare said text string to the predefined patient identifier, and, if a match is not found, give an alarm.

The present invention also relates to a medication dispenser. The medication dispenser according to the invention comprises a system according to the invention for extracting information from the medication sachets.

The medication dispenser according to the invention is used for dispensing medications to a patient. The medications to be dispensed are packaged into medication sachets which are attached to each other so that they form a strip of medication sachets. Each medication sachet contains the medications that the patient should take at the prescribed date and time.

The medication dispenser may comprise a container into which a strip of medication sachets can be inserted. The strip can be conveyed from the container to an outlet of the medication dispenser by using a pair of rollers which are rotated in opposite directions by a motor. The medication dispenser may comprise a cutting device that is configured to separate the medication sachets from the strip one medication sachet at a time. The separated medication sachets are conveyed to the outlet from which the patient can take the medication sachets. The medication dispenser may comprise a control device that is connected to the motor, the cutting device, and the processing device. The control device is configured to receive from the processing device the information extracted from the images of the medication sachets and to control the motor and the cutting device by utilising this information.

An advantage of the medication dispenser according to the invention is that the medication dispenser can dispense medications without knowing the layout according to which the information has been printed on the medication sachets.

The exemplary embodiments of the invention presented in this text are not interpreted to pose limitations to the applicability of the appended claims. The verb "to comprise" is used in this text as an open limitation that does not exclude the existence of also unrecited features. The features recited in the dependent claims are mutually freely combinable unless otherwise explicitly stated.

The exemplary embodiments presented in this text and their advantages relate by applicable parts to the method as well as the system according to the invention, even though this is not always separately mentioned.

### BRIEF DESCRIPTION OF THE DRAWINGS

- Fig. 1: illustrates a flow diagram of a method according to an embodiment of the invention,
- fig. 2: illustrates the processing of an image of an exemplary first medication sachet,
- fig. 3: illustrates the processing of an image of an exemplary second medication sachet, and
- fig. 4: illustrates a medication dispenser according to an embodiment of the invention.

### DETAILED DESCRIPTION OF THE DRAWINGS

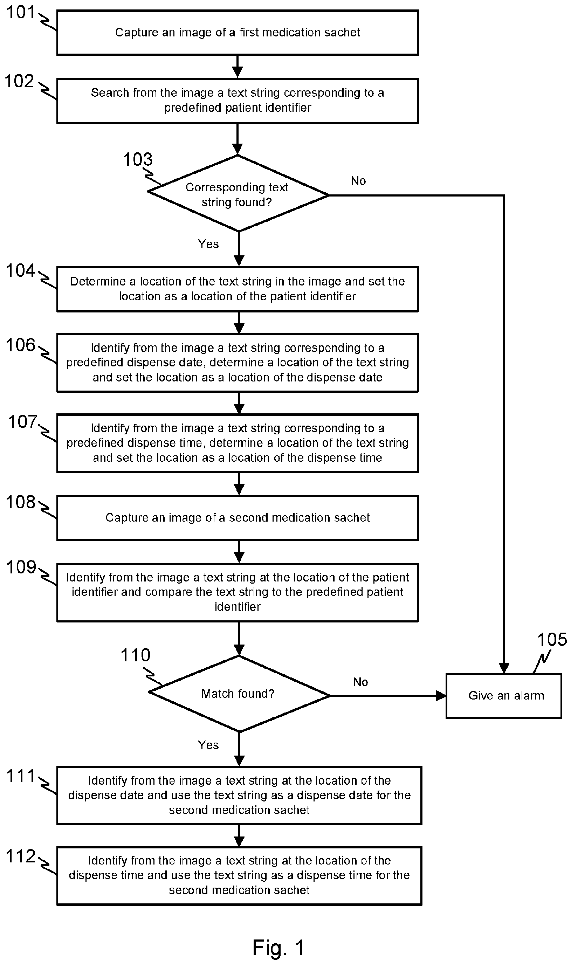

Fig. 1 illustrates a flow diagram of a method according to an embodiment of the invention for extracting information from medication sachets. The method can be used in a medication dispenser that utilises the information printed on the medication sachets for the purpose of dispensing medications to a patient.

The method begins at step 101, where an image of a first medication sachet is captured.

At step 102, a text string corresponding to a predefined patient identifier is searched from the image of the first medication sachet. At step 103, it is determined whether the predefined patient identifier is found from the image. If a corresponding text string is found, the method is continued at step 104. If a corresponding text string is not found, an alarm is given at step 105.

At step 104, a location of the text string corresponding to the predefined patient identifier is determined in the image of the first medication sachet and said location is set as a location of the patient identifier.

At step 106, a text string corresponding to a predefined dispense date of the first medication sachet is identified from the image of the first medication sachet, a location of said text string in the image of the first medication sachet is determined and said location is set as a location of the dispense date.

At step 107, a text string corresponding to a predefined dispense time of the first medication sachet is identified from the image of the first medication sachet, a location of said text string in the image of the first medication sachet is determined and said location is set as a location of the dispense time.

After the first medication sachet has been processed, the method continues with the processing of a second medication sachet at step 108, where an image of the second medication sachet is captured.

At step 109, a text string at the location of the patient identifier is identified from the image of the second medication sachet and said text string is compared to the predefined patient identifier.

At step 110, it is determined whether the text string matches with the predefined patient identifier. If a match is not found, an alarm is given at step 105. If a match is found, the method is continued at step 111.

At step 111, a text string at the location of the dispense date is identified from the image of the second medication sachet and said text string is used as a dispense date for the second medication sachet.

At step 112, a text string at the location of the dispense time is identified from the image of the second medication sachet and said text string is used as a dispense time for the second medication sachet.

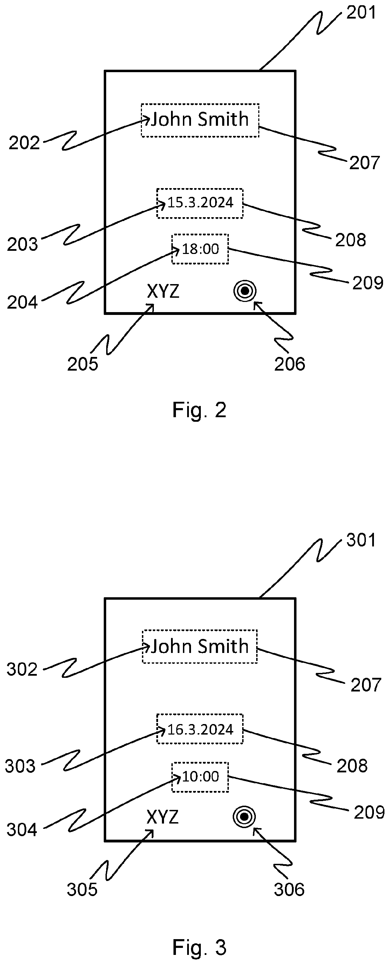

Fig. 2 illustrates the processing of an image of an exemplary first medication sachet 201. The first medication sachet 201 is provided with a patient identifier (i.e. patient's name) 202 as well as dispense date 203 and dispense time 204 of the medications inside the first medication sachet 201. The first medication sachet 201 is also provided with a pharmacy name 205 and company logo 206. The information has been printed using a specific layout on a surface of the first medication sachet 201.

Referring to the steps 102-104 in the flow diagram of fig. 1, the processing of the image includes searching from the image a text string corresponding to a predefined patient identifier, and assuming that the patient identifier 202 corresponds to the predefined patient identifier, determining a location of the patient identifier 202 and setting said location as a location 207 of the patient identifier.

Referring to the step 106 in the flow diagram of fig. 1, the processing of the image includes identifying from the image a text string corresponding to a predefined dispense date of the first medication sachet, and assuming that the dispense date 203 corresponds to the predefined dispense date, determining a location of the dispense date 203 and setting said location as a location 208 of the dispense date.

Referring to the step 107 in the flow diagram of fig. 1, the processing of the image includes identifying from the image a text string corresponding to a predefined dispense time of the first medication sachet, and assuming that the dispense time 204 corresponds to the predefined dispense time, determining a location of the dispense time 204 and setting said location as a location 209 of the dispense time.

The first medication sachet can be dispensed to a patient according to the predefined dispense date and time.

Fig. 3 illustrates the processing of an image of an exemplary second medication sachet 301. The second medication sachet 301 is provided with a patient identifier (i.e. patient's name) 302 as well as dispense date 303 and dispense time 304 of the medications inside the second medication sachet 301. The second medication sachet 301 is also provided with a pharmacy name 305 and company logo 306. The information has been printed using a specific layout on a surface of the second medication sachet 301.

Referring to the step 109 in the flow diagram of fig. 1, the processing of the image includes identifying from the image the patient identifier 302 at the location 207 of the patient identifier and comparing it to the predefined patient identifier to verify that the second medication sachet 301 is meant for the patient.

Referring to the step 111 in the flow diagram of fig. 1, the processing of the image includes identifying from the image the dispense date 303 at the location 208 of the dispense date and using it as a dispense date for the second medication sachet 301.

Referring to the step 112 in the flow diagram of fig. 1, the processing of the image includes identifying from the image the dispense date 304 at the location 209 of the dispense time and using it as a dispense time for the second medication sachet 301.

The second medication sachet 302 can be dispensed to a patient according to the dispense date 303 and dispense time 304 determined from the image of the second medication sachet 301.

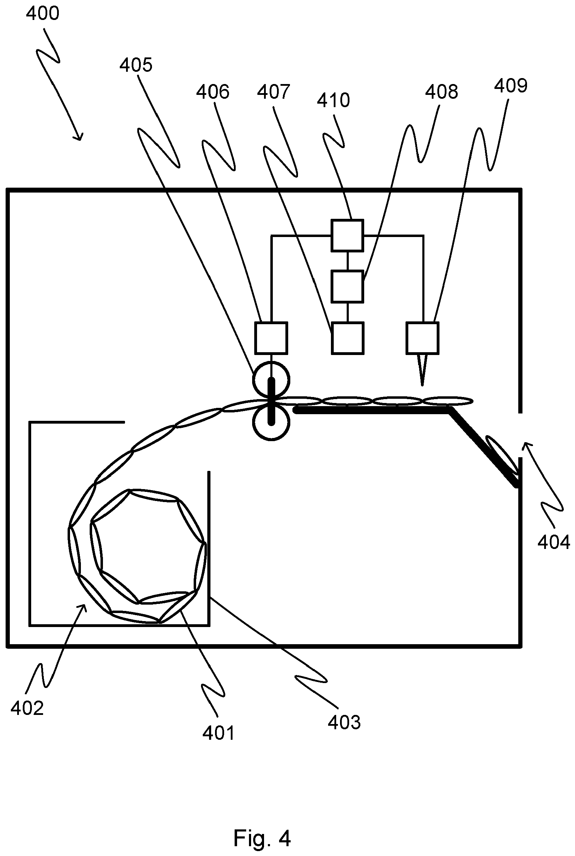

Fig. 4 illustrates a medication dispenser 400 according to an embodiment of the invention for dispensing medications to a patient. The medications to be dispensed have been packaged into medication sachets 401 which are attached to each other so that they form a strip 402 of medication sachets 401. Each medication sachet 401 contains the medications that the patient should take at the prescribed date and time. The medication dispenser 400 separates the medication sachets 401 from the strip 402 and delivers them to the patient one medication sachet 401 at a time.

The medication dispenser 400 comprises a container 403 into which the strip 402 of medication sachets 401 has been inserted. The strip 402 is conveyed from the container 403 to an outlet 404 of the medication dispenser 400 by using a pair of rollers 405 which are rotated in opposite directions by a motor 406.

The medication dispenser 400 comprises an imaging device 407 for capturing images of the medication sachets 401, and a processing device 408 that is connected to the imaging device 407 to receive the images from the imaging device 407. The processing device 408 is configured to extract information from the images of the medication sachets by using the method according to the invention. The information extracted from the images contains at least the dispense date and time of the medication sachets 401, which information is used for the purpose of dispensing medications to the patient.

The medication dispenser 400 comprises a cutting device 409 that is configured to separate the medication sachets 401 from the strip 402 one medication sachet 401 at a time. The separated medication sachets 401 are conveyed to the outlet 404 from which the patient can take the medication sachets 401.

The medication dispenser 400 comprises a control device 410 that is connected to the motor 406, the cutting device 409, and the processing device 408. The control device 410 is configured to receive from the processing device 408 the information extracted from the images of the medication sachets 401 and to control the motor 406 and the cutting device 409 by utilising this information.

Only advantageous exemplary embodiments of the invention are described in the figures. It is clear to a person skilled in the art that the invention is not restricted only to the examples presented above, but the invention may vary within the limits of the claims presented hereafter. Some possible embodiments of the invention are described in the dependent claims, and they are not to be considered to restrict the scope of protection of the invention as such.

## Claims

1. A method for extracting information from medication sachets, comprising:
- capturing an image of a first medication sachet,
**characterised in that** the method comprises:
- identifying from the image of the first medication sachet a text string corresponding to a predefined dispense date of the first medication sachet, determining a location of said text string in the image of the first medication sachet and setting said location as a location of the dispense date,
- identifying from the image of the first medication sachet a text string corresponding to a predefined dispense time of the first medication sachet, determining a location of said text string in the image of the first medication sachet and setting said location as a location of the dispense time,
- capturing an image of a second medication sachet,
- identifying from the image of the second medication sachet a text string at the location of the dispense date and using said text string as a dispense date for the second medication sachet, and
- identifying from the image of the second medication sachet a text string at the location of the dispense time and using said text string as a dispense time for the second medication sachet.

2. The method according to claim 1, **characterised in that** the method comprises searching from the image of the first medication sachet a text string corresponding to a predefined patient identifier.

3. The method according to claim 2, **characterised in that** the method comprises, if a text string corresponding to the predefined patient identifier is not found, giving an alarm.

4. The method according to claim 2, **characterised in that** the method comprises, if a text string corresponding to the predefined patient identifier is found, determining a location of said text string in the image of the first medication sachet and setting said location as a location of the patient identifier.

5. The method according to claim 4, **characterised in that** the method comprises identifying from the image of the second medication sachet a text string at the location of the patient identifier, comparing said text string to the predefined patient identifier, and, if a match is not found, giving an alarm.

6. The method according to any of the preceding claims, **characterised in that** the steps of the method are carried out in a medication dispenser.

7. The method according to claim 6, **characterised in that** the method comprises transmitting from the medication dispenser to another medication dispenser one or more of the following types of information: the location of the dispense date, the location of the dispense time, and the location of the patient identifier.

8. A system for extracting information from medication sachets, comprising:
- an imaging device configured to capture an image of a first medication sachet and an image of a second medication sachet,
**characterised in that** the system comprises:
- a processing device configured to:
- receive the image of the first medication sachet and the image of the second medication sachet from the imaging device,
- identify from the image of the first medication sachet a text string corresponding to a predefined dispense date of the first medication sachet, determine a location of said text string in the image of the first medication sachet and set said location as a location of the dispense date,
- identify from the image of the first medication sachet a text string corresponding to a predefined dispense time of the first medication sachet, determine a location of said text string in the image of the first medication sachet and set said location as a location of the dispense time,
- identify from the image of the second medication sachet a text string at the location of the dispense date and use said text string as a dispense date for the second medication sachet, and
- identify from the image of the second medication sachet a text string at the location of the dispense time and use said text string as a dispense time for the second medication sachet.

9. The system according to claim 8, **characterised in that** the processing device is configured to search from the image of the first medication sachet a text string corresponding to a predefined patient identifier.

10. The system according to claim 9, **characterised in that** the processing device is configured to, if a text string corresponding to the predefined patient identifier is not found, give an alarm.

11. The system according to claim 9, **characterised in that** the processing device is configured to, if a text string corresponding to the predefined patient identifier is found, determine a location of said text string in the image of the first medication sachet and set said location as a location of the patient identifier.

12. The system according to claim 11, **characterised in that** the processing device is configured to identify from the image of the second medication sachet a text string at the location of the patient identifier, compare said text string to the predefined patient identifier, and, if a match is not found, give an alarm.

13. A medication dispenser, **characterised in that** the medication dispenser comprises a system according to any one of claims 8 to 12.
